(19) Europäisches Patentamt
European Patent Office
Office européen des brevets

(11) Publication number: **0 121 964**
**B1**

(12) # EUROPEAN PATENT SPECIFICATION

(45) Date of publication of patent specification: **30.12.86**

(21) Application number: **84200325.3**

(22) Date of filing: **07.03.84**

(51) Int. Cl.[4]: **C 07 C 143/34,**
C 07 C 139/14, E 21 B 43/22,
C 10 G 1/04, C 09 K 3/00

(54) **Process for preparing an alkyl aryl sulphonate concentrate composition.**

(30) Priority: **08.04.83 GB 8309632**
**19.08.83 GB 8322423**

(43) Date of publication of application:
**17.10.84 Bulletin 84/42**

(45) Publication of the grant of the patent:
**30.12.86 Bulletin 86/52**

(84) Designated Contracting States:
**AT BE DE FR IT NL**

(56) References cited:
**EP-A-0 000 264**
**EP-A-0 016 357**
**DE-A-2 447 492**
**US-A-3 769 209**
**US-A-3 799 263**
**US-A-3 861 466**
**US-A-4 022 699**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Fernley, George William**
**Badhuisweg 3**
**NL-1031 CM Amsterdam (NL)**
Inventor: **Daane-Pluim, Gerdina Johanna Reinia**
**Badhuisweg 3**
**NL-1031 CM Amsterdam (NL)**
Inventor: **Verkouw, Hendrik Tijmen**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 121 964 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for preparing an alkyl aryl sulphonate concentrate composition, to compositions obtained thereby, and to their use in enhanced oil recovery processes.

The use of alkyl aryl sulphonates in enhanced oil recovery techniques is well known. US Patent Nos. 3,799,263 and 3,861,466 describe the use of alkyl xylene sulphonates, more particularly $C_{6-20}$ alkyl orthoxylene sulphonates, in enhanced oil recovery processes. In such processes the alkyl aryl sulphonates are typically incorporated at low concentration, e.g. of the order of 1% w/w, in a brine for injection into an oil-bearing formation, or may be used in soluble oil compositions which are injected into a reservoir prior to water-flooding (e.g. as described in US Patent No. 4,022,699).

In general, suitable brine and oil, in the form of crude oil, will be available at the point of use. It is therefore desirable to bring the alkyl aryl sulphonate to the point of use in as concentrated a form possible consistent with convenience of handling. Alkyl aryl sulphonates, such as sodium alkyl xylene sulphonates, are themselves of a thick, paste-like consistency, which tends to made them inconvenient to transfer from one container to another and gives rise generally to problems of transport and handling. Dilution of the alkyl aryl sulphonates with materials other than those which need themselves to be transported to the point of use, for inclusion with the alkyl aryl sulphonates in the enhanced oil recovery process, necessarily adds to transport costs and to increased material costs.

US Patent No. 4,022,699 discloses soluble oil compositions containing alkyl aryl sulphonates, specifically in the form of petroleum sulphonate, together with a stabilising agent, which may be *inter alia* an aliphatic alcohol. These compositons are prepared by admixing the alkyl aryl sulphonates (part of which are already in the form of a solution in oil) and the stabilising agent in at least 35%v of a liquid hydrocarbon (gas oil, gasoline or crude petroleum). There are also disclosed soluble oil additive concentrates for subsequent dilution with the liquid hydrocarbon, prepared by admixing the alkyl aryl sulphonates with the stabilising agent. However, in the only example described of such a concentrate (Example 16), the stabilising agent is ethylene glycol monobutyl ether and the alkyl aryl sulphonates employed are oil-containing solutions of petroleum sulphonates such that the final concentrate contains more than 20%v of oil.

US Patent No. 3,769,209, and the corresponding Canadian Patent No. 1,011,215 also discloses a soluble oil additive concentrate prepared by admixing alkyl aryl sulphonates with secondary butyl alhohol (s-butanol). However, in all the examples the alkyl aryl sulphonates, which are petroleum sulphonates, are employed in the form of oil-containing solutions such that the final concentrate contains more than 19%v of oil.

Surprisingly there has now been discovered a readily effected process by which a flowable alkyl aryl sulphonate concentrate composition may be prepared, which process does not depend upon substantial quantities of oil being present in the final composition.

According to the present invention there is provided a process for preparing an alkyl aryl sulphonate concentrate composition by neutralising at least one alkyl aryl sulphonic acid of general formula

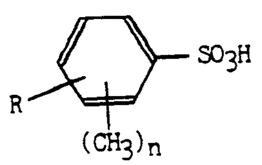

(I)

where n is 1 or 2 and R is a $C_{8-18}$ alkyl group, with an equivalent amount of a neutralising agent, which process comprises the steps of

a) mixing an aqueous solution containing at least 10% w/w of neutralising agent with at least one $C_{2-9}$ saturated alcohol, and

b) mixing the resulting mixture with the alkyl aryl sulphonic acid of formula I, the relative quantities of the aqueous solution and the at least one alcohol being such that the resulting neutralised mixture contains 5 to 40 parts by weight of the at least one alcohol per 100 parts by weight of alkyrl aryl sulphonate salt.

The acids of formula I are advantageously $C_{8-18}$ alkyl xylene sulphonic acids, e.d. n in formula I is 2, and R in formula I is preferably a $C_{8-16}$ alkyl group, more preferably a $C_{11-16}$ alkyl group. Those skilled in the art will appreciate that choice of an optimal alkyl aryl sulphonic acid for enhanced oil recovery is dependent *inter alia* on salinity levels in the particular oil bearing formation concerned.

The neutralising agent may conveniently be an ammonium or, preferably, an alkali metal hydroxide. Sodium hydroxide has been found to be very satisfactory.

The upper limit on the concentration of neutralising agent in the aqueous solution is determined by the solubility of the agent. If desired a saturate solution may be employed.

The concentration of alkyl aryl sulphonate salt and the at least one alcohol in the neutralised mixture from step b) will be dependent upon the neutralising agent employed and its concentration in the aqueous solution; however in general the alkyl aryl sulphonate concentration will be found to be in the range 53 to

89% w/w and the concentration of the at east one alcohol will be in the range 2.65 to 27% w/w, the balance being water and minor amounts of incidental components. Preferably the aqueous solution contains at least 20% w/w, more preferably at least 30% w/w, and advantageously 40 to 50% w/w, of the neutralising agent.

The at least one $C_{2-9}$ saturated alcohol is preferably a $C_{3-9}$ alkanol such as isopropanol, s-butanol, n-butanol, isobutanol, t-butanol, t-amyl alcohol, methyl isobutyl carbinol, 2-ethylhexanol or a mixture of $C_{3-9}$ alkanols, e.g. a mixture of $C_{7-9}$ primary alcohols such as that sold by members of the Royal Dutch/Shell group of companies under the designation "LINEVOL 79" (registered trade mark). Thus step a) in the process of the invention preferably comprises mixing the aqueous solution with at least one $C_{3-9}$ alkanol. S-butanol, especially, has been found to give very acceptable results.

The process of the invention may be effected over a wide range of temperatures, e.g. 10°C to 80°C. thus components from stock, at temperatures e.g. from 10°C to 40°C, may be used, or the alkyl aryl sulphonic acids may be used directly after production, when their temperatures are commonly in the range 55°C to 75°C. The process of the invention may thus conveniently be effected at ambient temperatures of at least 10°C.

Steps a) and b) may be carried out batchwise or continuously. In a batch process the order of mixing of the components in step a) is immaterial, but in step b) the alkyl aryl sulphonic acid should be added to the resulting mixture from step a), or the acid and the mixture should be simultaneously streamed into a mixing vessel, in order to minimise exposure of the alcohol component or components of the mixture to the unneutralised acid.

In accordance with a preferred embodiment of the invention the process includes the further step c) of mixing the neutralised mixture from step b) with up to 100 parts by weight, advantageously 5 to 40 parts by weight based on the alkyl aryl sulphonate sal of a cosurfactant selected from one or more alcohol ethoxy sulphates, alcohol ethoxylates, alcohol ethoxy acetates, alcohol ethoxy sulphonates and alpha-olefin sulphonates. Preferably the surfactant is at least one alcohol ethoxy sulphate. The cosurfactant preferably has average molecular weight in the range 300 to 750, more preferably 370 to 450. When the cosurfactant is an alpha-olefin sulphonate, it preferably contains 8—26 carbon atoms.

The invention also infludes the use of an alkyl aryl sulphonate concentrate composition whenever prepared by the process of the invention, in an enhanced oil recovery process.

Alkyl aryl sulphonate concentrate compositions prepared by the process of the invention have been found to be flowable, easily handled liquids.

The invention will be further understood from the following illustrative Examples.

Examples 1 to 3

Compositions were prepared by mixing 18 parts by weight s-butanol and sufficient quantities of aqueous solutions of sodium hydroxide (sodium hydroxide concentrations 46% w/w and 24% w/w) to neutralise 100 parts by weight of an alkyl-o-xylene sulphonic acid blend (containing 87% w/w pure acid) having average equivalent weight 400 and wherein the alkyl moiety contains 11 to 16 carbon atoms. To each of the resulting s-butanol/hydroxide mixtures at ambient temperature (20°C) was added with stirring 100 parts by weight of the alkyl-o-xylene sulphonic acid.

the resulting compositions were clear, golden, mobile liquids. To 94 parts by weight of the composition prepared using aqueous hydroxide of concentration 46% w/w was added 6 parts by weight of an alcohol ethoxy-sulphate ("DOBANOL 25—35/60") (registered trade mark) containing 58—60% active matter of average molecular weight 441 derived from $C_{12-15}$ alcohols and containing on average 3 ethoxy moieties per molecule.

For comparative purposes a similar procedure to the above was carried out using an aqueous sodium hydroxide solution of concentration 15% w/w.

The constitutions of the resulting compositions are given in Table I following, together with their viscosities, measured at 20°C using a "HAAKE ROTOVISCO" (trade mark) viscometer

TABLE I

| | Example | | | |
|---|---|---|---|---|
| | 1 | 2 | 3 | Comparative |
| % wt alkyl xylene sulphonate, sodium salts (AXS) | 65 | 57 | 61 | 48 |
| % wt s-butanol (parts per 100 parts AXS | 13(20) | 11(19) | 12(20) | 10(21) |
| % wt alcohol ethoxy sulphate (parts per 100 parts AXS) | — | — | 6(10) | — |
| % wt water (including minor amounts of incidental components) | 22 | 32 | 21 | 42 |
| Viscosity, m Pa.s at: 90 $s^{-1}$ | 1200 | 650 | 900 | * |
| 220 $s^{-1}$ | 1200 | 650 | 900 | * |
| 350 $s^{-1}$ | 1100 | 635 | 860 | * |
| 440 $s^{-1}$ | 1060 | 625 | 830 | * |

* not measurable; too viscous, non-homogeneous product.

Examples 4 to 6

Further compositions were prepared following a similar procedure to that of Examples 1 and 2, using a similar alkyl-o-xylene sulphonic acid blend (containing 98% w/w pure acid) having average equivalent weight 400 and wherein the alkyl moiety contains 11 to 16 carbon atoms, and aqueous sodium hydroxide solution of concentration 46% w/w, but employing different quantities of s-butanol. The constitutions of the resulting compositions, which were clear, golden, mobile liquids are given in Table II following together with their viscosities, measured at 40°C using a "HAAKE ROTOVISCO" (trade mark) viscometer.

TABLE II

| | Example | | |
|---|---|---|---|
| | 4 | 5 | 6 |
| % wt alkyl xylene sulphonate, sodium salt (AXS) | 75 | 72 | 70 |
| % wt s-butanol (parts per 100 parts AXS) | 11(15) | 14(19) | 17(24) |
| % wt water (including minor amounts of incidental components) | 14 | 14 | 13 |
| Viscosity, m Pa.s at: 100 $s^{-1}$ | 480 | 335 | 240 |
| 200 $s^{-1}$ | 480 | 340 | 240 |
| 300 $s^{-1}$ | 480 | 340 | 235 |
| 500 $s^{-1}$ | 470 | 335 | 225 |
| 700 $s^{-1}$ | 460 | 320 | 215 |

Examples 7 and 8

A composition was prepared following a procedure similar to that of Examples 1 and 2, using a similar alkyl-o-xylene sulphonic acid blend to those used in Examples 1 to 6 except that it contained 96% w/w pure acid, and a sodium hydroxide solution of concentration 46% w/w. To 93 parts by weight of the resulting

composition was added 7 parts by weight of the alcohol ethoxy sulphate used in Example 3.

The viscosities of the compositions, which were clear, golden, mobile liquids, were measured at 20°C and 40°C using a "HAAKE ROTOVISCO" (trade mark) viscometer, results being given in Table III following.

TABLE III

| | | Example | |
|---|---|---|---|
| | | 7 | 8 |
| % wt alkyl xylene sulphonate, sodium salt (AXS) | | 71 | 66 |
| % wt s-butanol (parts per 100 parts AXS) | | 14(20) | 13(20) |
| % wt alcohol ethoxy sulphate (parts per 100 parts AXS) | | — | 7(10) |
| % wt water (including minor amounts of incidental components | | 15 | 14 |
| Viscosity, m Pa.s at: | 20°C, 100 $s^{-1}$ | 1050 | 830 |
| | 200 $s^{-1}$ | 1030 | 795 |
| | 300 $s^{-1}$ | 930 | 785 |
| | 40°C, 100 $s^{-1}$ | 280 | 205 |
| | 200 $s^{-1}$ | 280 | 220 |
| | 300 $s^{-1}$ | 275 | 215 |

Examples 9 to 17

Following the procedures of Examples 3 and 8, using a similar alkyl-o-xylene sulphonic acid blend to those used in Examples 1 to 8 except that it contained 97% pure acid, a sodium hydroxide solution of concentration 46% w/w, the same alcohol ethoxysulphate and a variety of different alcohols, there were prepared a number of compositions of the following general constitution:

| | |
|---|---|
| alkyl xylene sulphonate, sodium salt (AXS) | 57% wt |
| alcohol (parts per 100 parts AXS) | 12% wt (21) |
| alcohol ethoxy sulphate (parts per 100 parts AXS) | 18% wt (32) |
| water (including minor amounts of incidental components) | 13% wt |

The compositions were all clear, golden mobile liquids.

The alcohols used, together with viscosities of the resulting compositions measured using a Ubbelohde viscometer at 20°C and 40°C, are given in Table IV following:

TABLE IV

| Example | Alcohol | Viscosity in mm²/s 20°C | Viscosity in mm²/s 40°C |
|---|---|---|---|
| 9 | isopropanol | 270 | 90 |
| 10 | s-butanol | 435 | 140 |
| 11 | n-butanol | 525 | 165 |
| 12 | isobutanol | 560 | 175 |
| 13 | t-butanol | 525 | 160 |
| 14 | t-amyl alcohol | 700 | 210 |
| 15 | methyl isobutyl carbinol | 945 | 260 |
| 16 | 2-ethylhexanol | 1715 | 450 |
| 17 | "LINEVOL 79" (registered trade mark) | 1665 | 455 |

"LINEVOL 79" is a mixture of $C_{7-9}$ primary alcohols, specific gravity at 20°C, 0.832.

The compositions of Examples 9 to 14 were subsequently stored at 10°C and remained liquid indefinitely.

Example 18

The composition was prepared according to the procedure of Example 8 using the same alkyl xylene sulphonic acid, s-butanol, a sodium hydroxide solution of concentration 46% w/w and the same alcohol ethoxy sulphate. Samples of the resulting composition, which was a clear, golden, mobile liquid were diluted with progressively larger amounts of water and the physical state of the resulting mixtures was observed. Results are given in Table V following, sample *a* being the undiluted composition.

TABLE V

| Sample | a | b | c | d | e | f | g | h | i |
|---|---|---|---|---|---|---|---|---|---|
| % wt alkyl xylene sulphonate, sodium salt (AXS) | 66 | 63 | 55 | 47 | 40 | 32 | 24 | 16 | 8 |
| % wt s-butanol (parts per 100 parts AXS) | 13(20) | 12 | 11 | 10 | 8 | 6 | 5 | 3 | 2 |
| % wt alcohol ethoxy sulphate (parts per 100 parts AXS) | 7(10) | 7 | 6 | 5 | 4 | 3 | 3 | 2 | 1 |
| % wt water | 14 | 17 | 28 | 38 | 48 | 59 | 68 | 79 | 89 |
| Physical state | mobile liquid | | | very viscous liquid or gel | | | | | mobile liquid |

Example 19

34 parts by weight s-butanol were mixed with 25 parts by weight of an aqueous solution of sodium hydroxide (sodium hydroxide concentration 46% w/w). To the resulting s-butanol/hydroxide mixture at ambient temperature (20°C) was added with stirring 100 parts by weight of an alkyl-o-xylene sulphonic acid blend (containing 94.2% w/w pure acid) having average equivalent weight 348 and wherein the alkyl moiety contains 8 to 14 carbon atoms. To 74 parts by weight of the resulting composition (which was a clear amber mobile liquid) was added 26 parts by weight of an alpha-olefin sulphonate containing 40% active matter of average molecular weight 310 derived from $C_{14-16}$ alpha-olefins.

The constitution of the final composition is given in Table VI following, together with its viscosity measured using a Ubbelohde viscometer at 20°C and 40°C.

TABLE VI

| | |
|---|---|
| %wt alkyl xylene sulphonate, sodium salts (AXS) | 48 |
| %wt s-butanol (parts per 100 parts AXS) | 16 (33) |
| %wt alpha-olefin sulphonate (parts per 100 parts AXS) | 10 (21) |
| %wt water (including minor amounts of incidental components) | 26 |
| Viscosity in mm2/s 20°C | 160 |
| 40°C | 56 |

Comparative Example A

An attempt was made to prepare a composition similar to that product in Example 1 by taking 100 parts by weight of the alkyl-o-xylene sulphonic acid, adding 20 parts by weight of s-butanol and then neutralising with 46% w/w aqueous sodium hydroxide. The attempt failed. The acid dehydrated the s-butanol to butene.

Comparative Example B

100 parts by weight of the alkyl-o-xylene sulphonic acid used in Example 1 was neutralised with 46% w/w aqueous sodium hydroxide. To the resulting thick paste was added 20 parts by weight s-butanol. On simple stirring the mixture remained a thick, difficult to handle, paste. On heating to 70°C and after continued and prolonged vigorous stirring at that temperature the mixture became clear and mobile, and on cooling to ambient temperature remained a clear, golden, mobile liquid similar in appearance to the composition of Example 1.

Comparative Example C

A blend of 80 %w of a commercial petroleum sulphonate (containing 60% w/w acive matter of average equivalent weight 465, the balance being 14% w/w non-sulphonatable organic matter and 26% w/w water containing a minor amount of sodium sulphate, 12%w "LINEVOL 79" (registered trade mark) and 8%w of the alcohol ethoxy sulphate used in Example 3, which blend was a thick block treacly material at ambient temperature was found to exhibit the following viscosity characteristics, measurement being effected using a "FANN" (trade mark) viscometer (Table VII).

TABLE VII

| Temperature | Viscosity, m la.s at | |
|---|---|---|
| | 15 $s^{-1}$ | 1500 $s^{-1}$ |
| 20°C | 9500 | 8000 |
| 45°C | 1300 | 1250 |
| 70°C | 350 | 340 |

**Claims**

1. A process for preparing an alkyl aryl sulphonate concentrate composition by neutralising a least one alkyl aryl sulphonic acid of general formula

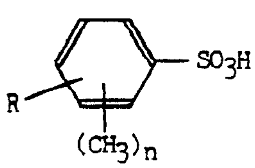

(I)

where n is 1 or 2 and R is a $C_{8-18}$ alkyl group, with an equivalent amount of a neutralising agent, which process comprises the steps of

a) mixing an aqueous solution containing at least 10% w/w of the neutralising agent with at least one $C_{2-9}$ saturated alcohol, and

b) mixing the resulting mixture with the alkyl aryl sulphonic acid of formula I, the relative quantities of the aqueous solution and the at least one alcohol being such that the resulting neutralised mixture contains 5 to 40 parts by weight of the at least one alcohol per 100 parts by weight of alkyl aryl sulphonate salt.

2. A process according to claim 1, which includes the further step

c) of mixing the neutralised mixture from step b) with up to 100 parts by weight based on the alkyl aryl sulphonate salt of a cosurfactant selected from one or more alcohol ethoxy sulphates, alcohol ethoxylates, alcohol ethoxy acetates, alcohol ethoxy sulphonates and alpha-olefin sulphonates.

3. A process according to claim 2, wherein the cosurfactant has average molecular weight in the range 300 to 750.

4. A process according to claim 3, wherein the cosurfactant has average molecular weight in the range 370 to 450.

5. A process according to any one of claims 1 to 4, wherein the neutralising agent is an alkali metal hydroxide.

6. A process according to claim 5, wherein the neutralising agent is sodium hydroxide.

7. A process according to any one of claims 1 to 6, wherein step a) comprises mixing the aqueous solution with at least one $C_{3-9}$ alkanol.

8. Use of an alkyl aryl sulphonate concentrate composition made by the process according to any one of the claims 1 to 7 in an enhanced oil recovery process.

**Patentansprüche**

1. Verfahren zur Herstellung einer konzentrierten Alkylarylsulfonatzusammensetzung durch Neutralisieren wenigstens einer Alkylarylsulfonsäure der allgemeinen Formel

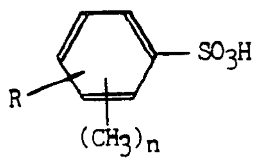

(I)

worin n den Wert 1 oder 2 aufweist und Reine $C_8$–$C_8$-Alkylgruppe bedeutet, mit einer äquivalenten Menge eines Neutralisationsmittels, welches Verfahren die Stufen

a) Vermischen einer wässerigen, wenigstens 10% Gew./Gew. des Neutralisationsmittels enthaltenden Lösung mit wenigstens einem $C_{2-9}$-gesättigten Alkohol, und

b) Vermischen des erhaltenen Gemisches mit der Alkylarylsulfonsäure der Formel I umfaßt, wobei die relativen Mengen der wässerigen Lösung und des wenigstens einen Alkohols derart sind, daß das erhaltene neutralisierte Gemisch 5 bis 40 Gewichtsteile des wenigstens einen Alkohols je 100 Gewichtsteile von Alkylarylsulfonatsalz enthält.

2. Verfahren nach Anspruch 1, welches die weitere Stufe

c) eines Vermischens des neutralisierten Gemisches aus Stufe b) mit bis zu 100 Gewichtsteilen, bezogen auf das Alkylarylsulfonatsalz, eines Co-Surfactants, ausgewählt unter einem oder mehreren Alkoholethoxysulfaten, Alkoholethoxylaten, Alkoholethoxyacetaten, Alkoholethoxysuylfonaten und α-Olefinsulfonaten, umfaßt.

3. Verfahren nach Anspurch 2, worin das Co-Surfactant ein mittleres Molekulargewicht im Bereich von 300 bis 750 aufweist.

4. Verfahren nach Anspruch 3, worin das Co-Surfactant ein mittleres Molekulargewicht im Bereich von 370 bis 450 aufweist.

5. Verfahren nach einem der Ansprüche 1 bis 4, worin das Neutralisationsmittel ein Alkalimetallhydroxid ist.

6. Verfahren nach Anspruch 5, worin das Neutralisationsmittel Natriumhydroxid ist.

7. Verfahren nach einem der Ansprüche 1 bis 6, worin die Stufe a) eine Vermischen der wässerigen Lösung mit wenigstens einem $C_{3-9}$-Alkanol umfaßt.

8. Verwendung einer konzentrierten Alkylarylsulfonatzusammensetzung, hergestellt nach dem Verfahren gemäß einem der Ansprüche 1 bis 7, in der Tertiärförderung.

**Revendications**

1. Un procédé pour la préparation d'une composition concentrée en alkylarylsulfonate en neutralisant au moins un acide alkylarylsulfonique de formule générale

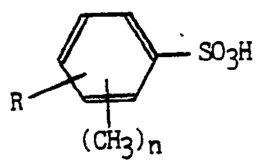

(I)

où n est 1 ou 2 et R est un groupe alkyle en $C_8$–$C_8$, avec une quantité d'un agent neutralisant, procédé qui comprend les étapes selon lesquelles

a) on mélange une solution aqueuse contenant au moins 10% en poids de l'agent de neutralisation avec au moins un alcool saturé en $C_{2-9}$ et

b) on mélange le mélange résultant avec l'acide alkylarylsulfonique de formule I, les quantités relatives de la solution aqueuse et de l'alcool ou des alcools étant telles que le mélange neutralisé résultant contienne 5 à 40 parties en poids de l'alcool ou des alcools pour 100 parities en poids de sel alkylarylsulonate.'

2. Un procédé selon la revendication 1, qui comprend l'étape supplémentaire c) selon laquelle on mélange le mélange neutralisé résultant de l'étape b) avec jusqu'à 100 parties en poids, per rapport au sel alkylarylsulfonate, d'un agent tensio-actif associé choisi parmi un ou plusieurs éthoxysulfates d'alcools, éthoxylates d'alcools, éthoxy-acétates d'alcools, éthoxysulfonates d'alcools et sulfonates d'alpha-oléfines.

3. Un procédé selon la revendication 2, dans lequel l'agent tensio-actif associé a un poids moléculaire moyen compris entre 300 et 750.

4. Un procédé selon la revendication 3, dans lequel l'agent tensio-actif associé a un poids moléculaire moyen compris entre 370 et 450.

5. Un procédé selon l'une quelconque des revendications 1 à 4, dans lequel l'agent de neutralisation est un hydroxyde de métal alcalin.

6. Un procédé selon la revendication 5, dans lequel l'agent de neutralisation est de l'hydroxyde de sodium.

7. Un procédé selon l'une quelconque des revendications 1 à 6, dans lequel l'étape a) comprend l'opération de mélange de la solution aqueuse avec au moins un alcanol en $C_{3-9}$.

8. L'utilisation d'une composition concentratée en alkylarylsulfonate préparée par un procédé selon l'une quelconque des revendications 1 à 7 dans un procédé amélioré d'extraction du pétrole.